Europäisches Patentamt

European Patent Office

Office européen des brevets

(11) Veröffentlichungsnummer: **0 029 126**
A1

(12) # EUROPÄISCHE PATENTANMELDUNG

(21) Anmeldenummer: 80106367.8

(22) Anmeldetag: 20.10.80

(51) Int. Cl.³: **A 61 B 5/14**

(30) Priorität: **20.11.79 DE 2946680**

(43) Veröffentlichungstag der Anmeldung: **27.05.81**
**Patentblatt 81/21**

(84) Benannte Vertragsstaaten: **AT BE CH FR GB IT LI LU NL SE**

(71) Anmelder: **C.A. Greiner & Söhne GmbH & Co. KG, Galgenbergstrasse 9c, D-7440 Nürtingen (DE)**

(72) Erfinder: **Krieg, Karl, Panoramastrasse 31, D-7440 Nürtingen (DE)**

(74) Vertreter: **Dreiss, Uwe, Dr. jur. Dipl.-Ing. M.Sc et al, Patentanwälte Dreiss & Fuhlendorf Schickstrasse 2, D-7000 Stuttgart 1 (DE)**

(54) **Blutentnahmegerät.**

(57) Blutentnahmegerät mit einem Halter (1; 401), in den exzentrisch eine doppelendige hohle Nadel (7) eingesetzt ist und in den ein Blutprobenröhrchen (2) eingeschoben wird. Das Blutprobenröhrchen (2) wird durch eine Membran (18) mit Hilfe einer aufschraubbaren und mit einer Öffnung versehenen Kappe (14) verschlossen. Das in den Innenraum des Halters (1; 401) hineinragende Ende der Nadel (7) durchstößt die Membran (18). Die Nadel (7) ist in einem Verstellring (403) angeordnet, der in einer Öffnung (402) im Halter (1; 401) drehbar ist.

EP 0 029 126 A1

## Blutentnahmegerät

Die Erfindung betrifft ein Blutentnahmegerät mit einem hohlzylindrischen Halter, in dessen einem Ende exzentrisch eine doppelendige hohle Nadel eingesetzt und in dessen anderes offenes Ende ein Blutprobenröhrchen einschiebbar ist, das durch eine Membran mit Hilfe einer aufschraubbaren und mit einer Öffnung versehenen Kappe verschlossen ist und bei der das in den Innenraum des Halters hineinragende Ende der Nadel die Membran durchstößt. Ein derartiges Blutentnahmegerät ist in der europäischen Patentanmeldung 80100830.1 vom 20.2.1980 beschrieben.

Eine exzentrische Anordnung der Nadel, welche durch die Abdichtung des Röhrchens durch Schraubkappe und Membran ermöglicht wird, hat den Vorteil, daß ein Einstich in eine Körperstelle, aus der Blut entnommen werden soll (z.B. Vene), unter einem besonders spitzen Winkel möglich ist.

Andererseits bereitet dies die Schwierigkeit, daß stets dafür gesorgt sein muß, daß eine bestimmte Orientierung der äußeren Nadelspitze gegeben ist. Die schräge Anschlifffläche der Spitze der Nadel soll zur Achse des Halters hinzeigen oder, anders ausgedrückt, von der Oberfläche der Einstichstelle abgewandt sein. Dann kann man den Vorteil der Exzentrizität der Nadel wahrnehmen. Es ist jedoch fertigungstechnisch äußerst schwierig, dafür zu sorgen, daß das Gewinde der in den Halter einschraubbaren Nadelhalterung und das im Halter vorgesehene Gewinde stets derart angeordnet sind, daß sich bei festem Sitz der Nadel im Halter genau diese Orientierung ergibt. Insbesondere ist dies deshalb problematisch, weil der Halter ja mehrfach verwendet wird.

Es ist Aufgabe der Erfindung, ein Blutentnahmegerät der eingangs genannten Art zu schaffen, bei dem stets die gewünschte Orientierung der Nadelspitze einfach erreichbar ist.

Erfindungsgemäß wird diese Aufgabe dadurch gelöst, daß die Nadel in einem Verstellring angeordnet ist, der in einer Öffnung im Halter verdrehbar ist. Die Erfindung betrifft ferner verschiedene vorteilhafte Weiterbildungen.

Die Nadelhalterung mit der Nadel wird also nicht mehr direkt in den Halter, sondern in einen in dem Halter verdrehbaren Verstellring eingeschraubt. Es ist besonders einfach, durch einen einfachen Handgriff die gewünschte Orientierung zu erzielen.

Ein Ausführungsbeispiel der Erfindung wird im folgenden unter Bezugnahme auf die beigefügten Zeichnungen beschrieben. Es stellen dar:

Figur 1    einen Querschnitt durch eine Blutentnahmevorrichtung;

Figur 2    eine Vorderansicht des Blutentnahmegerätes nach Fig. 1;

Figur 3    einen Querschnitt durch ein Ausführungsbeispiel;

Figur 4    eine Vorderansicht des Halters nach Fig. 3;

Figur 5    einen teilweisen Längsschnitt durch das Ausführungsbeispiel nach Fig. 3 mit eingesetztem Blutprobenröhrchen;

Figur 6    eine schematische Darstellung der Blutentnahme.

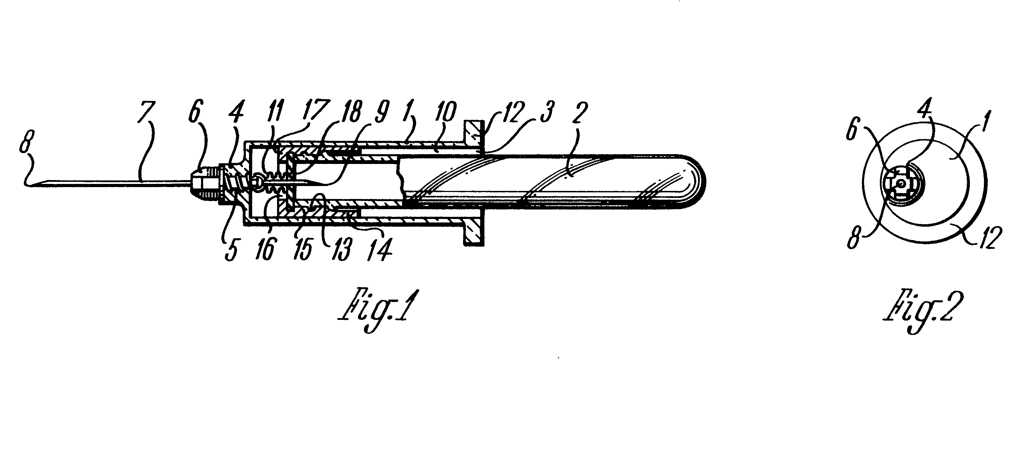

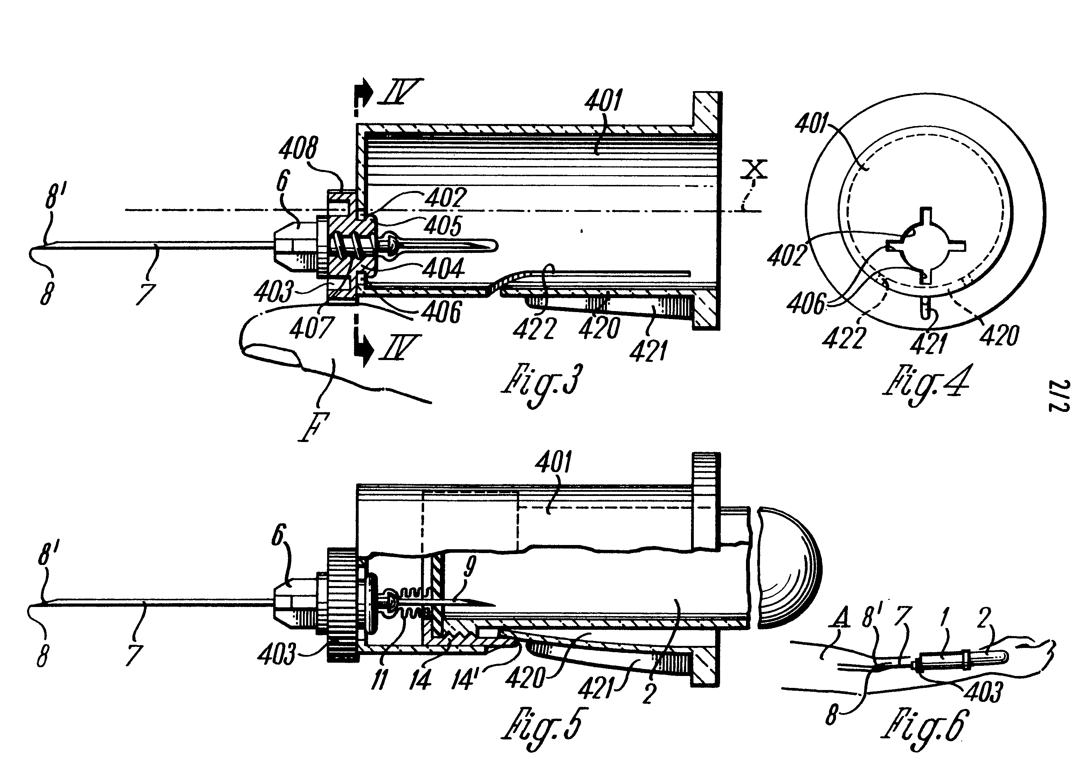

Das in den Fig. 1 und 2 dargestellte Blutentnahmegerät wird durch einen Halter 1 und einem Blutprobenröhrchen 2 gebildet. Der Halter 1 ist von hohlzylindrischer Form und an seinem einen Ende offen. An seinem anderen Ende 4 ist der Halter 1 mit einer Gewindebohrung 5 versehen, in die exzentrisch (siehe Fig. 2) eine Nadelhalterung 6 eingeschraubt ist. Die Nadelhalterung 6 dient zur festen Aufnahme einer doppelendigen hohlen Nadel 7, deren eines Ende 8 sich in der gezeigten Weise außerhalb des Halters 1 befindet und zur Blutentnahme in die Vene einer Person eingestochen wird (siehe Fig. 5), und deren anderes Ende 9 in das Innere des hohlzylindrischen Innenraums 10 des Halters 1 hineinragt. Der sich in den Innenraum 10 hineinerstreckende Teil der Nadel 7 ist von einer Schutzhülle 11 aus leicht durchstechbarem, leichtem, weichen Material umgeben. Am Ende 3 ist der Halter 1 mit einem Flansch 12 versehen, der die Handhabung bei der Blutentnahme erleichtert.

Das Blutprobenröhrchen 2, das vorzugsweise aus durchsichtigem Kunststoff hergestellt ist, weist einen Schraubverschluß auf. Dieser wird dadurch gebildet, daß auf das (in Fig. 1) linke und mit einem Schraubgewinde 13 versehene Ende des Blutprobenröhrchens 2

0029126

eine Kappe 14 aufgeschraubt wird, die mit einem entsprechenden Innengewinde 15 versehen ist. Die Kappe 14 weist eine Öffnung 16 auf, deren Durchmesser so bestimmt ist, daß der sich von außen nach innen und senkrecht zur Achse des Blutprobenröhrchens 2 erstreckende Rand 17 so breit ist, daß er eine Membran 18 gegen die offene Stirnseite des Blutprobenröhrchens 2 drückt. Dadurch wird das Innere des Blutprobenröhrchens 2 abgedichtet, so daß ein Vakuum im Röhrchen aufrecht erhalten werden kann.

Zur Blutentnahme nimmt man zunächst den Halter 1 mit der Nadel 7 in die Hand und sticht das Ende 8 in die Vene eines Patienten. Dann wird in den Halter 1 das kurzfristig vorher evakuierte Blutprobenröhrchen 2 eingeschoben, und zwar bis das innere Ende 9 der Nadel 7 die Membran 18 durchsticht. Dieser Zustand ist in Fig. 1 dargestellt. Dann saugt das Vakuum im Blutprobenröhrchen 2 Blut aus der Vene. Aus Fig. 2 ist ersichtlich, daß die Nadel 7 am Halter 1 exzentrisch angeordnet ist.

Beim Ausführungsbeispiel der vorliegenden Erfindung nach Fig. 3 bis 5 ist vorgesehen, daß die Nadel 7 bzw. die Nadelhalterung 6 so gedreht werden kann, daß die Spitze 8 der Nadel 7 stets die für einen Einstich optimale Orientierung annehmen kann.
Diese ist dann gegeben, wenn man, wie aus Fig. 5
ersichtlich, die exzentrische Anordnung der Nadel
im Halter dazu ausnützen kann, unter möglichst
spitzem Winkel zur Oberfläche der Körperstelle, an
der die Blutentnahme erfolgen soll, einen Einstich
durchzuführen. In Fig. 6 ist das am Beispiel des
Armes A eines Patienten dargestellt. Dazu ist dann
erforderlich, daß der Halter so gedreht wird, daß
die Nadel möglichst nahe an der Oberfläche dieser
Körperstelle liegt. Dann muß aber die Orientierung
der Nadel so sein, daß an der Spitze 8 die schräg
zur Achse unter spitzem Winkel erfolgte Anschlifffläche 8' zur Achse X des Halters 1 orientiert ist
(siehe Fig. 3) bzw. vom Körper wegzeigt. Nur ist
es schwierig, das Gewinde der Gewindebohrung 5 sowie das Außengewinde an der Nadelhalterung so zu bestimmen, daß stets eine solche Orientierung der Anschlifffläche 8' der Spitze 8 der Nadel 7 erfolgt,
insbesondere wenn - wie ja vorgesehen - der Halter 1
mehrfach verwendet wird.

Um diese Schwierigkeit zu überwinden, sieht das Ausführungsbeispiel nach Fig. 3 und 4 vor, daß in eine
Öffnung 402 im Halter 401 ein drehbarer Verstellring 403 eingesetzt ist. Der Verstellring 403 hat eine Nabe 404, deren Ende mit einem Klemmwulst 405 versehen ist. Da die Öffnung 402 mit Schlitzen 406 versehen ist, ist ein Eindrücken des Verstellringes 405 in die Öffnung 402 möglich (alternativ hierzu kann man auch vorsehen, daß Schlitze in der Nabe 404 vorgesehen sind, um deren Verformbarkeit durch Zusammendrücken beim Einpressen in die Öffnung 402 zu ermöglichen). In dem Verstellring 403 ist eine Gewindebohrung 407 vorgesehen, in welchen die Nadelhalterung eingeschraubt wird. Der Verstellring 403 ist entlang seines äußeren Umfangs mit einer Riffelung 408 versehen. Um den Verstellring leicht drehen zu können, z.B. mit einem Finger F, erstreckt sich der Rand des Verstellringes 403, wie aus Fig. 3 ersichtlich, mindestens bis zum Rand des Halters 1. Vorzugsweise steht er noch etwas über.

Ist die Nadelhalterung 6 in den Verstellring 403 eingeschraubt, so kann man durch Drehung des Verstellringes 403 die gewünschte Orientierung der schrägen Anschliffläche 8' der Nadelspitze 8 herbeiführen. Die Öffnung 402 ist so bemessen, daß eine leichte Klemmung des Verstellringes 403 im eingesetzten Zustand erfolgt; d.h.: einerseits ist eine Verdrehung möglich, andererseits ist die Klemmung stark genug, um den Ring 403 in der eingestellten Position sicher zu halten.

Fig. 3 zeigt ferner, daß in den Halter 401 ein Schlitz 422 eingeschnitten ist. Dadurch entsteht im Halter 401 eine Zunge 420. Wenn das Blutprobenröhrchen 2 in den Halter 401 eingeschoben ist, wird die Zunge 420 durch Handdruck etwas einwärts gedrückt. Sie greift damit hinter den Rand der Schraubkappe 14 und verhindert, daß das Blutprobenröhrchen 2 durch die elastische Rückdruckkraft der Schutzhülle 11 aus dem Halter 401 wieder herausgedrückt werden kann. Mit der Zunge 420 kann man also das Blutprobenröhrchen 2 im Halter 401 verriegeln. Um diese Verriegelung zu lösen, schiebt man das Blutprobenröhrchen 2 wieder im Halter 401 etwas weiter nach vorne. Dann federt die Zunge 420 wieder in ihre Ausgangslage (Fig. 3) zurück. Das Röhrchen kann dann ohne Hindernis aus dem Halter 401 herausgezogen werden. Um die Rückfederkraft dieser Zunge 420 zu erhöhen, ist an ihr eine Verstärkungsrippe 421 vorgesehen. Um leichter zu gewährleisten, daß die (in Fig. 5: linke) Vorderseite der Zunge 420 hinter den Rand der Schraubkappe 14 greift, ist dieser, wie bei 14' zu ersehen, abgerundet.

- Ende der Beschreibung -

0029126

- 1 -

Patentansprüche:

1. Blutentnahmegerät mit einem hohlzylindrischen Halter, in dessen einem Ende exzentrisch eine doppelendige hohle Nadel eingesetzt und in dessen anderes offenes Ende ein Blutprobenröhrchen einschiebbar ist, das durch eine Membran mit Hilfe einer aufschraubbaren und mit einer Öffnung versehenen Kappe verschlossen ist und bei der das in den Innenraum des Halters hineinragende Ende der Nadel die Membran durchstößt, dadurch gekennzeichnet, daß die Nadel (7) in einem Verstellring (403) angeordnet ist, der in einer Öffnung (402) im Halter (401) drehbar ist.

2. Blutentnahmegerät nach Anspruch 1, dadurch gekennzeichnet, daß der äußere Umfang des Verstellringes (403) den äußeren Rand des Halters (401) an einer Stelle übersteht.

0029126

3. Blutentnahmegerät nach Anspruch 1, dadurch gekennzeichnet, daß der Rand des Verstellringes (403) mit einer Riffelung (408) versehen ist.

4. Blutentnahmegerät nach Anspruch 1, dadurch gekennzeichnet, daß im Halter eine im entspannten Zustand das Einführen und Herausziehen des Blutentnahmeröhrchens (2) nicht behindernde Zunge (420) vorgesehen ist, die so weit elastisch einwärts in den Innenraum des Halters biegbar ist, daß sie hinter die Schraubkappe (14) eingreift und diese gegen ein Herausfallen sichert.

5. Blutentnahmegerät nach Anspruch 4, dadurch gekennzeichnet, daß die Zunge (420) durch einen Schlitz (422) im Halter (401) gebildet wird.

6. Blutentnahmegerät nach Anspruch 5, dadurch gekennzeichnet, daß die Zunge (420) durch eine Rippe (421) verstärkt ist.

*Fig.1*

*Fig.2*

*Fig.3*

*Fig.4*

*Fig.5*

*Fig.6*

Europäisches
Patentamt

**EUROPÄISCHER RECHERCHENBERICHT**

0029126

Nummer der Anmeldung

EP 80 10 6367

| | **EINSCHLÄGIGE DOKUMENTE** | | KLASSIFIKATION DER ANMELDUNG (Int. Cl.³) |
|---|---|---|---|
| Kategorie | Kennzeichnung des Dokuments mit Angabe, soweit erforderlich, der maßgeblichen Teile | betrifft Anspruch | |
| A | US - A - 4 166 450 (H.J.ABRAMSON/ METATECH CORP.)<br><br>* Zusammenfassung; Spalte 2, Zeilen 3-38; Spalte 3, Zeilen 5-56; Spalte 5, Zeilen 28-38 und Abbildungen 1-5 * | 1 | A 61 B 5/14 |
| | -- | | |
| | DE - C - 532 192 (SACHSISCHES SERUMWERK A.G.)<br><br>* Seite 2, Zeilen 30-37; und Zeilen 74-84; Seite 2, Zeile 121 - Seite 3, Zeile 15 und Abbildungen 1,2 * | 1 | RECHERCHIERTE SACHGEBIETE (Int. Cl.³)<br><br>A 61 B 5/14<br>A 61 M 5/315 |
| | -- | | |
| | US - A - 3 459 177 (F.DEUSCHLE/ BRUNSWICK CORP.)<br><br>* Spalte 3, Zeilen 3-60; Spalte 4, Zeilen 34-49 und Abbildungen 1-6 * | 2,3 | |
| | -- | | |
| | US - A - 3 513 829 (F.DEUSCHLE et al./BRUNSWICK CORP.)<br><br>* Spalte 3, Zeilen 25-62; Spalte 4, Zeilen 59-70; Spalte 5, Zeile 39 - Spalte 6, Zeile 21 und Abbildungen 6,8,9 * | 2,3 | KATEGORIE DER GENANNTEN DOKUMENTE<br><br>X: von besonderer Bedeutung<br>A: technologischer Hintergrund<br>O: nichtschriftliche Offenbarung<br>P: Zwischenliteratur<br>T: der Erfindung zugrunde liegende Theorien oder Grundsätze<br>E: kollidierende Anmeldung<br>D: in der Anmeldung angeführtes Dokument<br>L: aus andern Gründen angeführtes Dokument |
| | -- | | |
| | US - A - 3 181 529 (E.H.WILBURN)<br><br>* Spalte 11, Zeile 59 - Spalte 12, Zeile 16 und Abbildungen 22,23 * | 2,3 | |
| | --<br><br>./. | | |
| X | Der vorliegende Recherchenbericht wurde für alle Patentansprüche erstellt. | | &: Mitglied der gleichen Patent-familie, übereinstimmendes Dokument |

| Recherchenort | Abschlußdatum der Recherche | Prüfer |
|---|---|---|
| DEN HAAG | 26-02-1981 | RIEB |

EPA form 1503.1 06.78

Europäisches
Patentamt

| EINSCHLÄGIGE DOKUMENTE | | | KLASSIFIKATION DER ANMELDUNG (Int. Cl.³) |
|---|---|---|---|
| Kategorie | Kennzeichnung des Dokuments mit Angabe, soweit erforderlich, der Maßgeblichen Teile | betrifft Anspruch | |
| | US - A - 3 159 159 (M.J.COHEN)<br><br>* Spalte 5, Zeilen 38-67 und Abbildungen 5,6 *<br><br>-- | 4 | |
| | FR - A - 2 040 830 (P.BEN MOURA)<br><br>* Seite 4, Zeile 5 - Seite 5, Zeile 37 und Abbildungen 1-3 *<br><br>-- | 4,5 | |
| | FR - A - 1 384 386 (BECTON, DICKINSON AND COMPANY)<br><br>* Seite 1, rechte Spalte, Zeile 4 - Seite 2, rechte Spalte, Zeile 40 und Abbildungen 1-6 *<br><br>-- | 5 | **RECHERCHIERTE SACHGEBIETE (Int. Cl.³)** |
| P | DE - A - 2 908 817 (C.A.GREINER & SOHNE GMBH & CO.K.G.)<br><br>* Seite 7, Zeilen 10-25; Seite 10; Zeile 27 - Seite 11, Zeile 13; Seite 12, Zeilen 21-26; Abbildungen 1a, 4b und 6 * | 1 | |
| D,E | & EP - A - 0 017 728<br><br>------------- | | |